Europäisches Patentamt

European Patent Office

Office européen des brevets

(19)

(11) Publication number: **0 101 719**
**B1**

(12)

# EUROPEAN PATENT SPECIFICATION

(45) Date of publication of patent specification: 06.05.87

(21) Application number: 83901044.4

(22) Date of filing: 18.02.83

(86) International application number:
PCT/US83/00223

(87) International publication number:
WO 83/02940 01.09.83 Gazette 83/20

(51) Int. Cl.⁴: **C 07 C 51/14,** C 07 C 53/124,
C 07 C 67/38, C 07 C 69/24

(54) FORMATION OF ISOBUTYRIC ACID OR METHYL ISOBUTYRATE.

(30) Priority: 18.02.82 US 349720

(43) Date of publication of application:
07.03.84 Bulletin 84/10

(45) Publication of the grant of the patent:
06.05.87 Bulletin 87/19

(84) Designated Contracting States:
AT BE CH DE FR GB LI LU NL SE

(56) References cited:
EP-A-0 031 886
US-A-2 831 877
US-A-2 975 199
US-A-3 005 846
US-A-3 052 698
US-A-3 481 977
US-A-3 634 495
US-A-3 661 951
US-A-3 665 034
US-A-3 910 963
US-A-4 039 564

(73) Proprietor: **ASHLAND OIL, INC.**
P.O.Box 2219
Columbus OH 43216 (US)

(72) Inventor: **GROTE, Dace**
4973 Willoway Court East
Columbus, OH 43220 (US)

(74) Representative: **Weisert, Annekäte, Dipl.-Ing.
Dr.-Ing. et al**
Patentanwälte Kraus Weisert & Partner Thomas-
Wimmer-Ring 15
D-8000 München 22 (DE)

(56) References cited:
**Journal of Chemical Society (London), Perkin
Transactions I, issued 1979, B.L. Booth et al., A
comparison of the effectiveness of sulfuric acid
and trifluoromethanesulfonic acid in Koch
carboxylation reactions, pages 2441-2446, no.
10**

Note: Within nine months from the publication of the mention of the grant of the European patent, any person may
give notice to the European Patent Office of opposition to the European patent granted. Notice of opposition shall
be filed in a written reasoned statement. It shall not be deemed to have been filed until the opposition fee has been
paid. (Art. 99(1) European patent convention).

Courier Press, Leamington Spa, England.

## Description

The invention relates to a process for producing isobutyric acid or methyl isobutyrate by a continuous reaction of carbon monoxide, propylene and a hydroxy compound of water or methanol in the presence of liquid hydrogen fluoride, wherein the component of propylene is fed into the gas phase of the reacting mixture at substantially the rate at which reaction occurs.

EP—A—0,031,886, US—A—3,661,951, US—A—2,831,877 and US—A—3,005,846 describe a process for the preparation of isobutyric acid or the lower alkyl esters thereof by Koch synthesis, a method of carboxylating straight and branched aliphatic and alicyclic olefins with carbon monoxide, a process for the production of carboxylic acids comprising contacting an olefinic compound and carbon monoxide in liquid phase under substantially anhydrous conditions in the presence of a substantially water-free acid catalyst, or a process comprising reacting monoolefin and carbon monoxide in the presence of a mixture of HF and ethanol or water, respectively.

The prior art such as EP—A—0,031,886 on a whole is confusing because it shows the formation of oligomers, halogen substituted compounds, poor yields, self-esters, slow reaction times, or the necessity of low amounts of propylene and water during initial reaction to isobutyric acid or methyl isobutyrate from carbon monoxide, propylene, water, and/or methanol in the presence of hydrogen fluoride as well as the requirement for separation of excess water or methanol. These problems of the prior art are overcome by the process described herein.

The process of the present invention as defined above is characterized in that the residence time is from one tenth (0.1) to one-hundred-and-eighty (180) minutes, the reaction temperature is from thirty (30) to ninety (90) degrees Centigrade, the total reaction pressure is from one hundred (100) to four hundred (400) bar, the total mole ratio of anhydrous hydrogen fluoride to propylene in the reacting mixture is from ten (10) to fourty (40), the total mole ratio of hydroxy compound to reacted propylene is from nine-tenths (0.9) to nine hundred and ninety-nine thousands (0.999), and the total amount of reacted hydroxy compound is such that the final reaction mixture is substantially anhydrous and contains less than one hundred (100) parts per million of free hydroxy compound, based upon the amount of isobutyric acid or methyl isobutyrate, and removing the isobutyric acid or methyl isobutyrate from the final reaction mixture by simple distillation.

The carbon monoxide and propylene are fed into the rapidly mixing reaction mixture at substantially the same rate as the reaction between the reactants. For example, propylene is reacted with carbon monoxide in hydrogen fluoride and water or methanol wherein the final mole ratio of water to reacted propylene is from nine tenths (0.9) to nine hundred and ninety-nine thousandths (0.999) mole of water to one (1) mole of reacted propylene, at a temperature of from seventy four to eighty one (74—81°C) degrees Centigrade, a pressure of from one hundred and eighty six to two hundred (186—200) bars, and the isobutyric acid or its methyl ester is separated from the final anhydrous reaction mixture by removal of HF and IBF by simple distillation.

The invention concerns a novel and highly useful process for commercially preparing isobutyric acid or methyl isobutyrate, from propylene. The separated isobutyric acid or methyl isobutyrate can be oxydehydrogenated to methacrylic acid or methyl methacrylate.

Isobutyric acid or methyl isobutyrate is formed by reacting propylene with carbon monoxide and a hydroxy compound selected from water and methanol in the presence of hydrogen fluoride. The temperature of the reaction is maintained at from thirty (30) to ninety (90) degrees Centigrade, the total pressure is maintained at from one hundred (100) bars (1470 psi) to four hundred (400) bars (6000 psi), and the residence time is from one tenth (0.1) to one hundred and eighty (180) minutes. Propylene and carbon monoxide, either separately or mixed, are fed to the mixing reaction mixture at substantially the same rate as the reaction between the reactants. During the reaction, the mole ratio of carbon monoxide to propylene is determined by the pressure of carbon monoxide in the system, and the feed rate of the propylene. The mole ratio of hydrogen fluoride to propylene is within the range of from ten (10) to forty (40) moles of hydrogen fluoride to one (1) mole of propylene.

The total amount of hydroxy compound used is an amount whereby the final reaction mixture is anhydrous; that is, no free water or methanol is found in the final reaction mixture. The process further comprises the step of separating the reaction product of isobutyric acid or its corresponding methyl ester from the final reaction mixture by simple distillation of the HF and isobutyryl fluoride from the final product mixture. Because of the anhydrous nature of the final reaction mixture, the products are easily separated by standard procedures, for example, by distillation or other techniques known in the art.

In one embodiment of the invention, the reaction is conducted in the HF liquid phase which is presaturated with CO, and the carbon monoxide and propylene are fed into the vapor phase above the liquid phase, with the total amount of hydroxy compound being present in the liquid phase. The propylene and carbon monoxide are mixed separately or are premixed and the mixture of propylene and CO is fed into the vapor phase.

In another embodiment of the invention, the reaction is conducted in the carbon monoxide presaturated HF liquid phase, and the propylene and hydroxy compound, or propylene, carbon monoxide, and hydroxy compound are premixed and rapidly dispersed directly into the liquid phase of the reaction mixture.

In another embodiment of the invention, either propylene and hydroxy compound, or propylene, hydroxy compound, and carbon monoxide are premixed and fed into the vapor phase above the CO presaturated HF liquid phase of the reaction mixture.

When the hydroxy compound is fed simultaneously with the propylene or carbon monoxide or with both the propylene and carbon monoxide, the mole ratio of propylene to hydroxy compound being fed into the reacting mixture is from one and one hundredths (1.01) to one and one tenth (1.1) moles of propylene to 1.0 moles of hydroxy compound.

b. Reactants

The propylene may be from any source, and it may include inert material which does not interfere with the reaction, such as propane.

c. Reaction conditions

Although the total pressure of the reaction system during reaction will vary with the amount of reactants and temperature, the pressure range is generally from one hundred (100) bars to four hundred (400) bars (1470 psi to 6000 psi). The preferred pressure range for the process described herein is from one hundred (100) bars (1470 psi) to about two hundred (200) bars (3000 psi). The most preferred range is from 170 bars (2600 psi) to about 200 bars (3000 psi), particularly for the preferred temperature ranges.

Although the process temperature range may vary from thirty (30) degrees Centigrade (°C) to ninety (90) degrees Centigrade (°C), a preferred temperature range is from seventy (70) degrees Centigrade to ninety (90) degrees Centigrade.

The residence time may vary from one tenth (0.1) minute to one hundred and eighty (180) minutes depending upon the pressure and temperature. As the temperature and pressure increase, the residence time shortens, and at the preferred temperatures and pressures described herein, the residence time can be from one tenth (0.1) minute up to thirty (30) minutes. One of the critical features of the process described herein is that the reactants must be mixed; preferably, the faster the mixing the better the reaction, particularly under the preferred temperature, pressure conditions. The formation of self-esterification and oligomers has been a plague to the process, so that the carbonylation of propylene in hydrogen fluoride has not been feasible for commercial use, until the process described herein was discovered. Another critical feature of the process described herein is maintaining the amount of water or methanol, as described herein, below the stoichiometric amount of the final isobutyric acid, or its methyl isobutyrate ester, formed in the process; that is, the total amount of propylene reacted must be greater than the amount of isobutyric acid or methyl isobutyrate ester formed, such that the excess amount of propylene reacted is in the form of isobutyryl fluoride Thus, under the reaction conditions described herein, the water, or methanol, may be added to the anhydrous hydrogen fluoride prior to feeding of propylene and carbon monoxide. The amount of water or methanol initially added may be the total final amount to be reacted. Preferably the reaction mixture is saturated with carbon monoxide so that the reaction mixture contains a rapidly mixing mixture of water or methanol, hydrogen fluoride, and carbon monoxide. The carbon monoxide is dissolved up to its saturation point, and the excess pressure is mainly the vapor pressure of carbon monoxide above the liquid reaction mixture and maintains carbon monoxide saturation of the reaction mixture.

In the preferred process, propylene is added to the vapor or gas phase above the reacting CO saturated HF liquid mixture; that is, it is injected into the vapor phase which is comprised mainly of carbon monoxide. The rate of addition being substantially the same as the rate of the reaction of propylene with the reactants; that is, within eighty percent but preferably within ninety or more percent of the reaction rate.

In another embodiment, a small amount of the hydroxy compound can be added to the anhydrous hydrogen fluoride acid, for example from one hundredth (0.01) to about one (1) or two (2) percent or up to sixteen (16) percent by weight of the anhdyrous acid and the remaining amount can be injected by separate lines or by premixing the hydroxy compound (e.g., $H_2O$) with the propylene or carbon monoxide and injected into the vapor phase above the reacting mixture. However, the final reaction mixture is anhydrous, with respect to water or methanol. As used herein the term "anhydrous" means free from water and/or methanol; that is, the amount of water and/or methanol is less than 100 parts by million of the acid, and preferably less than 1 part by million; and it is highly preferred that the methanol and/or water is undetectable.

In general, the total amount of the hydroxy compound added is less than the amount required for the propylene to be completely converted under the reaction conditions to isobutyric acid (if water is used) or the methyl isobutyrate ester (if methanol is used); that is, the final reaction mixture prior to further reaction or separation is substantially anhydrous, as described herein. If methanol is used then the system is substantially free from methanol, as described herein.

In another embodiment, the propylene may be dispersed directly into the rapidly mixed reaction mixture. For example, it may be rapidly dispersed, as finely atomized droplets into a rapidly mixing CO, HF or CO, HF, $H_2O$ or $CH_3OH$ reaction mixture.

The amount of CO to propylene in the reaction mixture is determined by the CO pressure.

In general the mole ratio of hydrogen fluoride to propylene is from ten (10) moles to forty (40) moles of hydrogen fluoride to one (1) mole of propylene, a preferred range is from twelve (12) to eighteen (18) moles of hydrogen fluoride to one (1) mole of propylene.

In general the mole ratio of hydroxy compound to moles of reacted propylene is from nine hundred and ninety-nine thousandths (0.999) to nine tenths (0.9) moles of hydroxy compound to one (1) mole of reacted propylene, a preferred ratio is from nine hundred and ninety-nine thousandths (0.999) to ninety-five hundredths (0.95) moles of hydroxy compound to one (1) mole of reacted propylene.

In general the amount of water or methanol initially added to the hydrogen fluoride, may range from one thousandths (0.001) mole percent of the total amount of propylene to be reacted to nine hundred and ninety-nine thousandths (0.999) mole percent of the total amount of propylene to be reacted; provided that, the total initial amount of water and/or methanol is less than sixteen (16), generally less than ten (10) weight percent of hydrogen fluoride, and preferably to avoid corrosion, particularly with water less than five (5) weight percent of the hydrogen fluoride, and especially preferred as less than two (2) or one (1) weight percent of hydrogen fluoride. For example, if the total amount of propylene to be reacted is 1 mole (42 grams), the total amount of water used in the reaction would be up to 0.999 mole percent or 0.999 moles (0.999 moles × 18 grams per mole of water=17.982 grams of water). This total amount of water could be added initially to the hydrogen fluoride, provided that the total amount of water is less·than sixteen (16) preferably less than ten (10) or five (5) weight percent of the total amount of anhydrous hydrogen fluoride (HF). Thus, if the mole ratio of HF to propylene to be reacted is ten (10) moles to one (1) mole of propylene, then for one (1) mole of propylene to be reacted, the amount of HF is ten (10) moles (10 moles × 20 grams per mole HF=200 grams of HF), and the total amount of water that initially could be present is preferably 5 weight percent of the HF (0.05 × 200 grams = 10.0 grams of water). The remaining water would be to be added periodically or gradually at the same rate it is being used to keep the weight of water below or at five (5) weight percent, and less than the total amount of propylene reacted so that the excess propylene is in the form of isobutyryl fluoride. When all of the propylene reacts, the total amount of water will have been used up, and the system will be anhydrous, containing isobutyric acid, isobutyryl fluoride, carbon monoxide (dissolved in the HF) and hydrogen fluoride (and possibly very small amounts of some higher weight acids and/or oligomers, but no self esters).

If the mole ratio of HF to propylene is forty (40) moles to one (1) mole of propylene to be reacted, then for one (1) mole of propylene to be reacted, the amount of HF is forty (40) moles or (40×20=800 grams HF), and for the most preferred method only two (2) weight percent of water would be present or (800×.02=16.0 grams of water) would be added initially and the remainder added gradually or periodically, so that in the final reaction mixture after reaction is complete no detectable water exists, and only anhydrous HF, isobutyric acid (possibly very small amounts of higher acids or. oligomers), isobutyryl fluoride, and carbon monoxide are present.

The final reaction mixture, because it is anhydrous, is readily separated without expensive distillation equipment. For example, the HF or the HF and isobutyryl fluoride can readily be flashed off in a simple separation unit, or by using a one or two plate distillation column. The isobutyric acid, or the methyl isobutyrate is readily separated from any of the high boiling compounds, by simple distillation. The separated methyl isobutyrate or isobutyric acid is then readily deoxyhydrogenated over catalysts described and known to those skilled in the art to the corresponding methyl methacrylate or methacrylic acid.

Examples

The following Examples will illustrate the invention described herein:

Procedure

A Hastelloy C Autoclave Magnedrive reactor of three hundred cubic centimeters capacity was charged with a hydrofluoric acid solution (HF/$H_2O$) containing 94.6% by weight of hydrogen fluoride, (or a methanol-hydrogen fluoride solution), pressurized to the predetermined pressure with carbon monoxide and heated to the predetermined temperature, while the mixture was rapidly stirred. Propylene was injected at a predetermined rate and the moles of propylene reacted exceeded the moles of water or methanol present in the reactor, approximately 105 mole % of propylene based on moles of water. Carbon monoxide was also added to the rapidly stirring mixture, at about the same rate as the propylene reacts by monitoring the pressure drop of the reaction and adding carbon monoxide to maintain the predetermined pressure range.

Upon completion of the reaction, after pressure stabilization, the reactor was cooled and analyzed. In the case of the isobutyric acid samples, excess water was pumped into the reactor, and the reactor was vented, disassembled and the product was analyzed after further dilution with ice (until a 10% hydrofluoric acid—90% water solution forms), addition of $Na_2SO_4$, and extraction with cyclohexane. The cyclohexane extracts were analyzed by gas chromatography for yields of isobutyric acid, and verified by the distillation yield. Distillation residues were analyzed for higher molecular weight acids and oligomers content as a check on the propylene mass added.

Example I

The above described procedure was followed using 8.55 g (0.475 moles) water, 151.5 g. (7.58 moles) of anhydrous hydrogen fluoride. The total amount of propylene added was 20.9 grams (0.50 moles) giving a final HF/propylene/$H_2O$ mole ratio of 15.2/1.0/0.95 molar. The temperature range was 29 to 33°C, the pressure range was 2690—2800 psig (184 to 192 bars), the stirring rate was 1450 to 1550 revolutions per minute, the total time for addition of propylene was 20.5 minutes, the rate of propylene addition was approximately 0.19 moles of propylene per mole of hydrogen fluoride per hour. The apparent reaction time (inclusive of the propylene addition time) was from 59 to 90 minutes. The yields of products obtained were 41.6 grams of isobutyric acid (based on distillation analysis, 95% yield of isobutyric acid), 0.2 grams of residue, (higher boiling residues than isobutyric acid). Total relationship of propylene by analysis is 20.1 grams compared to weight amount added 20.9 grams (96% accountability) no self ester products formed.

Column 1 of Table I gives the cumulative pressure lost (pressure change) in pounds per square inch (psig) versus time.

TABLE I
(Example I)

| Cumulative pressure loss (psi) | Time in minutes | |
|---|---|---|
| −10 | 1.5 | |
| −10 | 3.5 | |
| 20 | 5.5 | |
| 80 | 7.5 | |
| 110 | 9.5 | |
| 130 | 11.5 | |
| 170 | 13.5 | |
| 200 | 15 | |
| 240 | 17 | |
| 260 | 18.5 | |
| 280 | 19.5 | |
| 290 | 20.5 | propylene pump shut off |
| 350 | 22.5 | |
| 390 | 24.5 | |
| 460 | 26.5 | |
| 490 | 30.5 | |
| 580 | 41.0 | |
| 670 | 51.5 | |
| 710 | 59.5 | |
| 720 | 66.5 | |
| 770 | 80.5 | |
| 790 | 92.5 | |
| 790 | 97.5 | |

Example II

The procedure described above was followed, while maintaining a temperature range of 26 to 32°C, a pressure range of 2730 to 2930 psig (186—200 bars), and a stirring rate of 1800—1750 revolutions per minute. The initial HF/propylene (to be reacted) $H_2O$ molar ratio was 15/1/0.95, the total charge of propylene was 21.1 grams, the propylene to HF molar rate of addition per hour was 0.26, and the apparent reaction time (inclusive of the propylene addition time) to point of pressure stabilization was 88 minutes. The propylene was added in 15.5 minutes. The yield of isobutyric acid was 95 percent (41.4 grams) with 0.4 grams as high boiling residues and no self esters.

Example III

The above described procedure was followed, while maintaining the temperature within the range of 77—87°C, the pressure range from 2668—2860 psig (181—194 bars), the stirring rate at 2000 rpm. The HF/propylene/$H_2O$ molar charge ratio was 15.7/1/.99, the total charge of propylene was 19.7 grams, the propylene to HF molar rate of addition per hour was 0.16, the propylene addition time was 24.25 minutes, and the apparent reaction time (inclusive of the propylene addition time) until pressure stabilization was 34 minutes. Under these conditions, the yield of isobutyric acid based on distillation was 96%, the accountability of propylene was 98%, 0.5 grams of oligomers were found and no self esters.

Example IV

The described procedure was followed, while maintaining the temperature within the range of seventy-four (74) to eighty-one (81) degrees Centigrade, the pressure range from 2711—2860 psig (186—194 bars), and the stirring rate at 2000 rpm. The initial HF/propylene (to be reacted)/$H_2O$ molar ratio was 15.0/1/0.95, the total charge of propylene was 20.8 grams, the propylene to HF molar rate of addition per hour was 0.15, the propylene addition time was 27 minutes, and the apparent reaction time until pressure stabilization was 29 minutes (this is inclusive of the propylene addition time). Under these conditions the yield of isobutyric acid based on distillation was 98% with no self esters, with a 100% accountability.

The yield of IBA based on gas chromatography analysis was 100%.

Comparative Example

The following example shows the formation of isopropyl isobutyrate by self-esterification.

The described procedure as used in Example I was followed, while maintaining the temperature within the range of twenty-six (26) to twenty-nine (29) degrees Centigrade, but the pressure range was from sixty one (61) bars (900 psi) to seventy one (71) bars (1043 psi), and the stirring rate was 2100 rpm. The initial HF/propylene (to be reacted)/$H_2O$ molar ratio was 15/1/0.95, the total charge of propylene was 20.6 grams, and the propylene to HF molar rate of addition per hour was 0.14. The propylene addition time was 29.5 minutes, and the apparent reaction time (which includes the propylene addition time) until pressure stabilization was 100 minutes. Under these conditions the yield of isobutyric acid based on distillation was only 82%, with an accountability of 94%, but 1.53 grams of isopropyl isobutyrate formed (5% selectivity based on propylene) due to self-esterification.

Example V

The procedure described above was followed using 149 gram (7.4 moles) of anhydrous hydrogen fluoride to which 15.3 grams (0.47 moles) of anhydrous methanol was added. The total amount of propylene added was 21 grams (0.5 moles), which gave us a final HF/propylene/methanol mole ratio of 14.8/1.0/0.94. The temperature range was from 29°C to 33°C, the pressure range was from 2796 psig (191 bars) to 2840 psig (194 bars), the stirring rate was 1450 to 1500 revolutions per minute. The time for addition of propylene was 38 minutes, and the rate of propylene addition was approximately 0.11 moles of propylene per mole of hydrogen fluoride per hour. The apparent reaction time (inclusive of the propylene addition time) was about 80 to 90 minutes. The yield of methyl isobutyrate was about 97%, with no formation of dimethyl ether, acetic acid or methyl acetate. The amount of high boiling materials was only 0.6 grams.

Example VI

The procedure of Example V was followed using 150 grams (7.4 moles) of anhydrous hydrogen fluoride to which 15.35 grams (0.48) moles of anhydrous methanol was added. The total amount of propylene added was 21.1 grams (0.50) moles, which gave a final HF/propylene/methanol mole ratio of 15/1/0.96. The temperature range was from 69 to 71°C, the pressure range was from 2722 psig (186 bars) to 2894 psig (198 bars) and the stirring rate was from 1450 to 1500 revolutions per minute. The total addition time for propylene was 38 minutes, and the rate of addition of propylene was 0.10 moles of propylene per one mole of anhydrous hydrogen fluoride per hour. The apparent reaction time (inclusive of the addition time of propylene) was about 44 minutes.

The yield of methyl isobutyrate was about 99%, with no formation of dimethyl ether, acetic acid, or methyl acetate. The amount of high boilers was only 0.25 grams.

**Claims**

1. A process for producing isobutyric acid or methyl isobutyrate by a continuous reaction of carbon monoxide, propylene and a hydroxy compound of water or methanol in the presence of liquid hydrogen fluoride, wherein the component of propylene is fed into the gas phase of the reacting mixture at substantially the rate at which reaction occurs, characterized in that the residence time is from one tenth (0.1) to one-hundred-and-eighty (180) minutes, the reaction temperature is from thirty (30) to ninety (90) degrees °C, the total reaction pressure is from one hundred (100) to four hundred (400) bar, the total mole ratio of anhydrous hydrogen fluoride to propylene in the reacting mixture is from ten (10) to fourty (40), the total mole ratio of hydroxy compound to reacted propylene is from nine-tenths (0.9) to nine hundred and ninety-nine thousands (0.999), and the total amount of reacted hydroxy compound is such that the final reaction mixture is substantially anhydrous and contains less than one hundred (100) parts per million of free hydroxy compound, based upon the amount of isobutyric acid or methyl isobutyrate, and removing the isobutyric acid or methyl isobutyrate from the final reaction mixture by simple distillation.

2. The process of Claim 1, characterized in that the total pressure of the reaction is from about one-hundred-and-seventy (170) bar to about two-hundred (200) bar.

3. The process of Claims 1 and 2, characterized in that the temperature of the reaction is from about seventy (70) to ninety (90) degrees Centigrade.

4. The process of Claims 1 to 3, characterized in that the total mole ratio of hydroxy compound to reacted propylene is from 0.95 to 0.999.

5. The process of Claims 1 to 4, characterized in that the hydrogen fluoride and the isobutyryl fluoride are flashed off and the isobutyric acid or methyl isobutyrate is removed by simple distillation.

**Patentansprüche**

1. Verfahren zur Herstellung von Isobuttersäure oder Methylisobutyrate durch kontinuierliche Umsetzung von Kohlenmonoxid, Propylen und einer Hydroxyverbindung von Wasser oder Methanol in Gegenwart von flüssigem Fluorwasserstoff, wobei die Propylenkomponente in die Gasphase des reagierenden Gemisches mit im wesentlichen der Geschwindigkeit eingespeist wird, mit der die Reaktion stattfindet, dadurch gekennzeichnet, daß die Verweilzeit ein Zahntel (0,1) bis einhundertachtzig (180) Minuten beträgt, daß die Reaktionstemperatur dreißig (30) bis neunzig (90) Grad Celsius (°C) beträgt, daß der Gesamtreaktionsdruck einhundert (100) bis vierhundert (400) bar beträgt, daß das Gesamtmolverhältnis von wasserfreiem Fluorwasserstoff zu Propylen in dem reagierenden Gemisch zehn (10) bis vierzig (40) beträgt, daß das Gesamtmolverhältnis von Hydroxyverbindung zu umgesetztem Propylen neun Zehntel (0,9) bis neunhundertneunundneunzig Tausendstel (0,999) beträgt und daß die Gesamtmenge von umgesetzter Hydroxyverbindung so ist, daß das Endreaktionsgemisch im wesentlichen wasserfrei ist und weniger als einhundert (100) Teile pro Million freie Hydroxyverbindung, bezogen auf die Menge von Isobuttersäure oder Methylisobutyrat, beträgt, und daß man die Isobuttersäure oder das Methylisobutyrat aus dem Endreaktionsgemisch durch einfache Destillation entfernt.

2. Verfahren nach Anspruch 1, dadurch gekennzeichnet, daß der Gesamtdruck der Reaktion etwa einhundertsiebzig (170) bar bis etwa zweihundert (200) bar beträgt.

3. Verfahren nach Anspruch 1 und 2, dadurch gekennzeichnet, daß die Reaktionstemperatur etwa siebzig (70) bis neunzig (90) Grad Celsius ist.

4. Verfahren nach den Ansprüchen 1 bis 3, dadurch gekennzeichnet, daß das Gesamtmolverhältnis von Hydroxyverbindung zu umgesetztem Propylen 0,95 bis 0,999 beträgt.

5. Verfahren nach den Ansprüchen 1 bis 4, dadurch gekennzeichnet, daß man den Fluorwasserstoff und das Isobutyrylfluorid schnell abdampft und daß man die Isobuttersäure oder das Methylisobutyrat durch einfache Destillation entfernt.

## Revendications

1. Procédé pour la production d'acide isobutryique ou d'isobutyrate de méthyle par une réaction continue de monoxyde de carbone, de propylène et d'un composé hydroxy de l'eau ou du méthanol en présence d'un fluorure d'hydrogène liquide, où le composé de propylène est introduit dans la phase gazeuse du mélange réactionnel à une vitesse substantiellement la même à laquelle se déroule la réaction, caractérisé en ce que le temps de contact est compris entre un dixième (0,1) et cent quatre-vingt (180) minutes, en ce que la température de la réaction est comprise entre trente (30) et quatre-vingt dix (90 degrés °C, en ce que la pression totale de la réaction est comprise entre cent (100) et quatre cent (400) bar, en ce que le rapport molaire total du fluorure d'hydrogène anhydre sur le propylène dans le mélange réactionnel est compris entre dix (10) et quarante (40), en ce que le rapport total molaire du composé hydroxy sur le propylène ayant réagi est comprise entre neuf dixièmes (0,9) et neuf cent quatre-vingt dix-neuf millièmes (0,999) et en ce que la quantité totale du composé hydroxy ayant réagi est telle que le mélange réactionnel final est pratiquement anhydre et contient moins de cent (100) parties par million de composé hydroxy libre, basé sur la quantité d'acide isobutyrique ou d'isobutyrate de méthyle, et élimination de l'acide butyrique ou de l'isobutyrate de méthyle du mélange réactionnel final par simple distillation.

2. Procédé selon la revendication 1, caractérisé en ce que la pression totale de la réaction est comprise entre cent soixante-dix (170) bar et deux cent (200) bar.

3. Procédé selon les revendications 1 et 2, caractérisé en ce que la température de la réaction est comprise entre soixante-dix (70) et quatre vingt-dix (90) degrés centigrade.

4. Procédé selon l'une des revendications 1 à 3, caractérisé en ce que le rapport molaire total du composé hydroxy sur le propylène ayant réagi est compris entre 0,95 et 0,999.

5. Procédé selon l'une des revendications 1 à 4, caractérisé en ce que le fluorure d'hydrogène et le fluorure d'isobutyryle sont séparés selon une méthode flash et l'arcade isobutyrique ou l'isobutyrat de méthyle est éliminé par simple distillation.